# EUROPEAN PATENT APPLICATION

(11) **EP 0 768 378 A1**
(43) Date of publication of application: **16.04.1997**
(21) Application number: 95922757.0
(22) Date of filing: 23.06.1995
(51) Int. Cl.: C12N 15/12, C12P 21/02, C07K 14/47, A61K 38/17

(54) **DNA CODING FOR CALDECRIN AND PROCESS FOR PRODUCING CALDECRIN BY USING THE DNA**

(30) Priority: 24.06.1994 JP 164898/94; 08.03.1995 JP 74676/95
(71) Applicant: Chugai Seiyaku Kabushiki Kaisha, Tokyo 115 (JP)
(72) Inventor: TOMOMURA, Akito, Kagoshima-shi Kagoshima 891-01 (JP); SAHEKI, Takeyori, Kagoshima 891-01 (JP); NOIKURA, Takenori, Kagoshima 890 (JP); AKIYAMA, Masashi Chugai Seiyaku Kabushiki Kaisha, Gotenba-shi Shizuoka 412 (JP)
(74) Representative: VOSSIUS & PARTNER
(86) International application number: JP9501268
(87) International publication number: WO9600287

(57) **Abstract**

The present invention provides a DNA coding for caldecrin and a process for producing caldecrin by using that DNA.

The present invention discloses a DNA coding for caldecrin with the specific amino acid sequences consisting of 239 amino acids shown in SEQ ID NO: 1 or 2, a DNA that hybridizes with said DNA, and a process for producing caldecrin by using a host that has been transformed by the introduction of said DNA.

## Description

### TECHNICAL FIELD

The present invention relates to a genetic engineering process for producing caldecrin which has the effect of lowering serum calcium concentration.

### BACKGROUND ART

In the past, since acute pancreatitis is accompanied by hypocalcemia, there was thought to be a factor present in pancreatitis that lowers serum calcium concentration. In addition, Takaoka, et al. reported that administration of porcine pancreas extract lowers serum calcium concentration, while the inventors of the present invention successfully purified the serum calcium-lowering factor, caldecrin, from porcine pancreas extract as one of its components (Japanese Unexamined Patent Publication No. 4-279598).

Pig caldecrin is a protein having a molecular weight of about 28,000 Da. It dose-dependently lowers serum calcium concentration in mice, and exhibits inhibitory activity against parathyroid hormone (PTH)-induced calcium release in the cultured fetal mouse bone system [Raisz, L.G., J. Clin. Invest., 44, 103-116 (1965)]. This indicates that caldecrin lowers serum calcium concentration as a result of inhibition of bone resorption by PTH in vivo.

In addition, caldecrin is homologous to serine proteases such as elastase and chymotrypsin, and although caldecrin itself has chymotrypsin-type serine protease activity, its mouse serum calcium-lowering effects and anti-PTH effects in cultured mouse fetal bone are not inhibited even when treated with phenylmethanesulfonyl fluoride (PMSF), an irreversible serine protease inhibitor. This suggests the possibility that caldecrin or its fragments in which protease activity has been inactivated continue to express serum calcium-lowering activity.

Moreover, the inventors of the present invention found a factor that possesses these properties in rat pancreas also, and following its isolation and purification (Japanese Patent Application No. 6-027578), reported on its physiological activity (12th Meeting of the Japan Bone Society (1994), 16th Meeting of the U.S. Bone Metabolism Society (1994), and 10th International Conference on Intracellular Protein Catabolism (1994)).

### DISCLOSURE OF THE INVENTION

The present invention is intended to provide a process that enables rat or human caldecrin to be produced in large amounts by a recombinant technology process. Moreover, the present invention is also intended to provide a DNA that codes for the caldecrin or its precursor as a means therefor.

Thus, the present invention provides a DNA coding for rat caldecrin comprising the amino acid sequence from the amino acid Val at position 1 to the amino acid Leu at position 239 of SEQ ID NO: 1, or its precursors. As one example, the present invention provides a DNA coding for rat caldecrin having the amino acid sequence from the amino acid Val at position 1 to the amino acid Leu at position 239 in SEQ ID NO: 1. Moreover, as another example, the present invention provides a DNA coding for a rat caldecrin precursor having the amino acid sequence from the amino acid Met at position -29 to the amino acid Leu at position 239 in SEQ ID NO: 1.

In addition, the present invention provides a DNA coding for human caldecrin comprising the amino acid sequence from the amino acid Val at position 1 to the amino acid Leu at position 239 in SEQ ID NO: 2, or its precursor. As one example, the present invention provides a DNA coding for human caldecrin having the amino acid sequence from the amino acid Val at position 1 to the amino acid Leu at position 239 in SEQ ID NO: 2. Moreover, as another example, the present invention provides a DNA coding for a human caldecrin precursor having the amino acid sequence from the amino acid Met at position -29 to the amino acid Leu at position 239 in SEQ ID NO: 2.

Moreover, the present invention provides an expression vector containing any of the various above-mentioned DNAs, a host transformed by said expression vector, and a process for producing human caldecrin or its precursor characterized by culturing of said host and recovering the human caldecrin or its precursor from that culture.

Moreover, the present invention provides a rat caldecrin precursor containing the amino acid sequence from the amino acid Val at position 1 to the amino acid Leu at position 239 in SEQ ID NO: 1.

Moreover, the present invention provides a human caldecrin precursor containing the amino acid sequence from the amino acid Val at position 1 to the amino acid Leu at position 239 in SEQ ID NO: 2.

### BRIEF DESCRIPTION OF THE DRAWINGS

Fig. 1 shows the method of sequencing the cDNA coding for rat caldecrin, and the positional relationship between the amino acid sequence of rat caldecrin as predicted from the nucleotide sequence of said cDNA and those of enzymatic degradation fragments of rat pancreatic caldecrin.

Fig. 2 shows the C18 reverse phase column chromatography elution profile of peptide fragments produced by digestion of caldecrin derived from rat pancreas with metalloendopeptidase (Grifola frondosa origin).

Fig. 3 shows the structure of an expression plasmid for the insertion of rat caldecrin DNA used in Example 3.

Fig. 4 shows the result of electrophoresis demonstrating the expression of rat caldecrin of the present invention in animal cells.

Fig. 5 indicates that the cDNA expression product of the present invention possesses the effect of inhibiting calcium release induced by PTH.

Fig. 6 shows the C18 reverse phase column chromatography elution profile of peptide fragments produced by digestion of caldecrin derived from pig pancreas with metalloendopeptidase of Grifola frondosa origin.

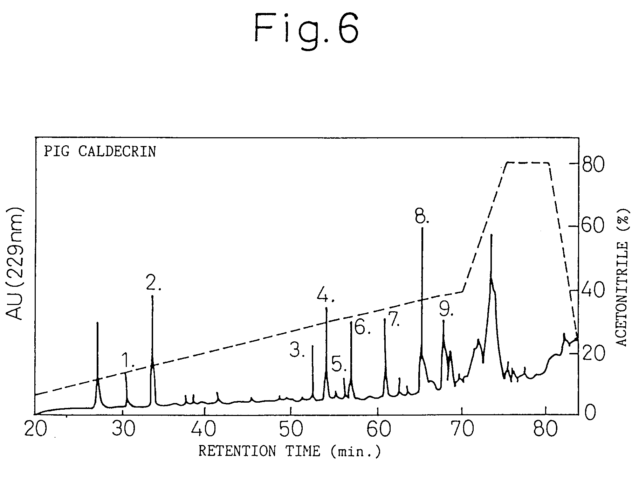

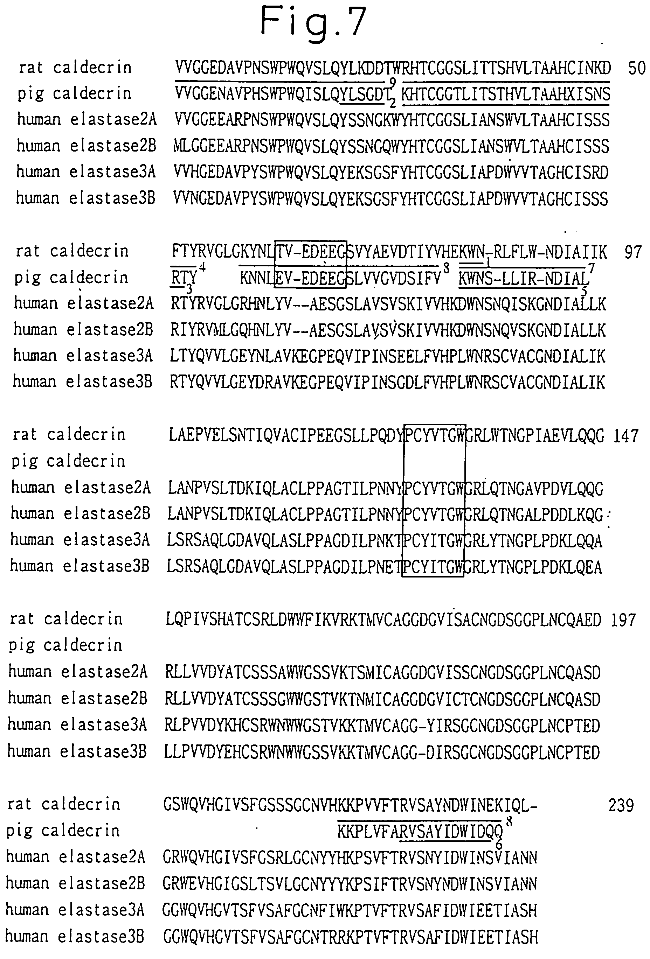

Fig. 7 indicates the alignment of the amino acid sequence predicted from the cDNA of rat caldecrin, with the amino acid sequences of the peptide fragments produced by digestion of caldecrin derived from pig pancreas with metalloendopeptidase of Grifola frondosa origin, and the amino acid sequences of human elastases. In the figure, the numbered straight lines indicate the amino acid sequences of the peptide fragments corresponding to the peak numbers in Fig. 6, the portions enclosed in boxes indicate caldecrin-specific sequences or sequences common to elastases including caldecrin, that correspond to oligonucleotides used as PCR primers in Example 7.

Fig. 8 indicates the process for construction, and the structure, of the transfer vectors pBPHC1 and pBPHC2 for expressing recombinant caldecrin using a Baculovirus expression system.

Fig. 9 shows the result of electrophoresis by Western blotting which demonstrates the expression of the human caldecrin of the present invention in insect cells.

In the figure, HC1 is a photograph of Western blotting in the case of using pBPHC1, while HC2 is a photograph of Western blotting in the case of using pBPHC2. Each medium was concentrated five fold by lyophilization, and 2 µl of the culture supernatant obtained in Example 9 was spotted on a 12.5% gel in lane 1, while 5 µl of said culture supernatant was spotted on said 12.5% gel in lane 2.

### DETAILED DESCRIPTION

The present inventors performed immunoscreening of a rat pancreas λgtllcDNA library using rabbit antisera obtained following immunization with pig caldecrin as antigen, prepared a DNA fragment from the resulting positive clone, and then used the DNA fragment as a probe to obtain the full-length rat caldecrin cDNA. Subsequently, this cDNA was inserted downstream of the β-actin promoter of the mammalian cell expression vector, pCAGGS, developed by Miyazaki, et al. [Niwa, H., Yamamura, K. and Miyazaki, J., Gene, 108, 193-200 (1991)] to construct the expression plasmid pCAGGS-Rat Caldecrin.

The BMT-10 cells developed by Gerard, R.D. et al. were transfected with this plasmid and recombinant rat caldecrin was produced by transient expression. Recombinant rat caldecrin, that was confirmed over time by Western blotting, was expressed in the culture supernatant of BMT-10 cells transfected with pCAGGS-Rat Caldecrin, after which the dose-dependent inhibitory activity against PTH-induced calcium release in the above-mentioned cultured fetal mouse bone system was confirmed, thereby leading to completion of the present invention.

The rat caldecrin cDNA cloned according to the present invention has the nucleotide sequence shown in SEQ ID NO: 1, and the amino acid sequence deduced from that nucleotide sequence is also shown in SEQ ID NO: 1. In this amino acid sequence, the sequence starting from the 30th amino acid Val (amino acid No. 1), from the N terminus is equivalent to the N-terminal amino acid sequence of rat caldecrin isolated from rat pancreas (Japanese Patent Application No. 6-027578). Thus, the amino acid sequence from the amino acid Val at position 1 to the amino acid Leu at position 239 of SEQ ID NO: 1 is predicted to be the amino acid sequence of mature rat caldecrin.

Thus, in one embodiment, the present invention provides a DNA coding for rat caldecrin comprising the amino acid sequence from the amino acid Val at position 1 to the amino acid Leu at position 239 in SEQ ID NO: 1, or its precursor. One embodiment thereof is a DNA coding for mature rat caldecrin having the amino acid sequence from Val at position 1 to the amino acid Leu at position 239 in SEQ ID NO: 1. Another embodiment provides a DNA coding for rat caldecrin precursor having the amino acid sequence from Met at position -29 to the amino acid Leu at position 239 in SEQ ID NO: 1.

However, precursor encoded by the DNA of the present invention is not limited to that described above, but rather includes DNAs coding for various lengths of the pre-form or pro-form.

An example of a nucleotide sequence of the DNA of the present invention is that which comprises the nucleotide sequence from the nucleotide G at position 88 to the nucleotide G at position 804 in SEQ ID NO: 1. A specific example of this is the DNA coding for mature rat caldecrin consisting of the base sequence from the nucleotide G at position 88 to the nucleotide G at position 804 in SEQ ID NO: 1.

Moreover, an example of another embodiment of the present invention is a DNA coding for a rat caldecrin precursor consisting of the nucleotide sequence from the nucleotide A at position 1 to the nucleotide G at position 804 in SEQ ID NO: 1. However, the nucleotide sequence of the DNA coding for the rat caldecrin precursor of the present invention is not limited to that described above, but rather includes DNA comprised of a corresponding part of the nucleotide sequence shown in SEQ ID NO: 1, coding for various lengths of the pre-form or pro-form.

The present inventors determined the partial amino acid sequence of caldecrin derived from pig pancreas by analyzing the amino acid sequences of pig caldecrin degradation fragments resulting from digestion with metalloendopeptidase of Grifola frondosa origin. The present inventors further found a sequence specific for caldecrin by aligning these amino acid sequences with the amino acid sequence of rat caldecrin cDNA which was already known, and the amino acid sequences of human elastases 2A, 2B, 3A and 3B.

Mixed oligonucleotides corresponding to this amino acid sequence and oligonucleotides corresponding to the DNA sequences commonly present in both the elastases and caldecrin were synthesized, and a polymerase chain reaction (PCR) was performed with Tag polymerase using these oligonucleotides as primers and a human pancreatic cDNA library as a template. Since analysis of an approximately 200 bp DNA sequence obtained from this reaction showed a high degree of homology with the DNA sequence encoding rat caldecrin, this DNA was considered to be a fragment of human caldecrin cDNA.

Next, using the resulting DNA fragment as a probe, hybridization screening was performed on a human pancreatic λgt11 cDNA library to allow determination of the human caldecrin cDNA sequence along with the full-length amino acid sequence inferred from the cDNA sequence as shown in SEQ ID NO: 2. The sequence starting with Val at position 30 from the N-terminus of this amino acid sequence corresponds to the mature sequences of rat caldecrin (Japanese Patent Application No. 6-027578 and Japanese Patent Application No. 6-164898) and pig caldecrin (Japanese Unexamined Patent Publication No. 4-279598), with the sequence from Met at position -29 to Ser at position -14 corresponding to the signal sequence (pre-sequence), and the sequence from Cys at position -13 to Arg at position -1 corresponding to the pro-sequence.

In addition, although a plurality of clones obtained by hybridization screening are not uniform, but rather have sequence diversity as shown in SEQ ID NO: 2, the resulting clones do not contain known cDNA sequences such as those elastase cDNA. This result supports the contention that the DNA fragment used for hybridization screening is a sequence specific to caldecrin.

Moreover, in order to produce human caldecrin by recombinant technology, the present inventors established a production process using insect cells and Baculovirus that expresses a large amount of nuclear polyhedra by specifically infecting those cells. Specifically, in order to remove non-coding DNA sequences at the 5' and 3' terminal ends of human caldecrin cDNA, a DNA sequence from the start codon to the stop codon was amplified by PCR, and the resulting fragment was inserted downstream of a polyhedrin promoter in the transfer vector pBacPAK9.

Next, when the transfer vector containing human caldecrin cDNA was transfected into host insect Sf9 cells together with a viral DNA, BacPAK6, which had been digested with Bsu36I and lost a portion of the gene essential for replication, homologous recombination occurred between the transfer vector and the virus DNA, and only infectious virus containing human caldecrin cDNA was amplified as a mature virus. When this virus was again used to infect Sf9 cells and the culture supernatant was applied to SDS-polyacrylamide gel electrophoresis (SDS-PAGE), a band corresponding to an approximately 30 kD expressed protein was observed.

Since this band reacted with anti-pig caldecrin antibody in Western blotting, it was considered to be human caldecrin containing a pro-sequence. As described above, a process for producing recombinant human caldecrin using recombinant technology techniques was established, thereby leading to completion of the present invention.

As one aspect, the present invention provides a DNA coding for human caldecrin comprising the amino acid sequence from the amino acid Val at position 1 to the amino acid Leu at position 239 in SEQ ID NO: 2, or its precursor. One embodiment of the present invention is a DNA coding for mature human caldecrin having the amino acid sequence from the amino acid Val at position 1 to the amino acid Leu at position 239 in SEQ ID NO: 2, while another embodiment is a DNA coding for a human caldecrin precursor having the amino acid sequence from the amino acid Met at position -29 to the amino acid Leu at position 239 in SEQ ID NO: 2.

However, precursors encoded by the DNA of the present invention are not limited to the above-mentioned precursor, but rather include DNAs coding for pre-forms or pro-forms of various lengths.

An example of a nucleotide sequence of the DNA of the present invention is that comprising the nucleotide sequence from the nucleotide G at position 88 to nucleotide G at position 804 in SEQ ID NO: 2. A specific example of this is the DNA coding for mature human caldecrin consisting of the base sequence from nucleotide G at position 88 to nucleotide G at position 804 in SEQ ID NO: 2.

Moreover, still another embodiment is exemplified by the DNA coding for a human caldecrin precursor consisting of the nucleotide sequence from the nucleotide A at position 1 to the nucleotide G at position 804 in SEQ ID NO: 2. However, the nucleoride sequence of the DNA coding for the human caldecrin precursor of the present invention is not limited to that described above, but, rather, the present invention also provides DNA containing a portion of the nucleotide sequence shown in SEQ ID NO: 2, coding for pre-forms and pro-forms of various lengths.

Moreover, although human caldecrin cDNA is not uniform, as is shown in SEQ ID NO: 2, the present invention shows that the sequence contained in the amino acid sequence from the amino acid Glu at position 63 to the amino acid Trp at position 131 in SEQ ID NO: 2 is specific for caldecrin. Consequently, the present invention provides a process for cloning various human caldecrin cDNAs by hybridization screening using as a probe a DNA comprising all or a portion of the nucleotide sequence corresponding to that amino acid sequence.

Thus, in addition to the DNA and amino acid sequences predicted from it shown in SEQ ID NO: 2, the present invention also includes DNAs in human cDNA or genome libraries and amino acid sequences corresponding to them, which DNAs hybridize to a DNA containing all or a portion of the nucleotide sequence corresponding to the amino acid sequence from the amino acid Glu at position 63 to the amino acid Trp at position 131 in SEQ ID NO: 2.

In addition, the present invention also provides a DNA that hybridizes to DNA having a nucleotide sequence shown in SEQ ID NO: 1 or 2 and codes for a protein having a serum calcium-lowering effect. In this case, examples of DNAs that hybridize to the above-mentioned DNA include the DNAs that hybridize to a labeled DNA probe prepared from the above-mentioned DNA by incubating for 15 to 16 hours at 40-45°C in 100 ml of a hybridization solution containing said labeled DNA probe, such as a hybridization solution containing 50 ml of formamide, 25 ml of 20x SSC, 10 ml of 50x Denhardt's solution, 1 ml of 10% SDS and 1 ml of 10 mg/ml denatured salmon sperm DNA.

The DNA of the present invention can be cloned by, for example, the hybridization screening described in Examples 1 and 8 or the PCR method described in Example 7. In addition, DNAs can also be chemically synthesized in accordance with routine methods. DNAs coding for mature human caldecrin or its precursors of various lengths of the present invention can be prepared by, for example, cleaving with restriction enzymes or shortening with an exonuclease the long DNA shown in SEQ ID NO: 1 or 2, or by inserting a translation start codon adjacent to and upstream of the N-terminus of a desired polypeptide.

In addition, the present invention also relates to an expression plasmid containing any of the above-mentioned DNAs. This plasmid contains a promoter for expressing said DNAs, an expression control sequence such as a terminator, and an origin of replication for replicating in a suitable host.

Moreover, the present invention relates to a host transformed by the above-mentioned expression vector and a process for producing human caldecrin or its precursor using said host.

The human caldecrin DNA of the present invention is not limited to application in the insect cell expression system described in Example 9, but can also be applied to other expression systems, and after insertion into various expression vectors, is able to produce recombinant human caldecrin in conventionally used hosts, including procaryotic cells such as Escherichia coli, lower eucaryotic cells such as yeast, or higher eucaryotic cells such as cultured mammalian cells.

Specific examples of hosts that can be used include Escherichia coli, Bacillus such as Bacillus subtilis, yeasts such as Saccharomyces, and molds including Aspergillus such as Aspergillus niger. Moreover, cultured mammalian cells such as cultured mouse, rat and human cells can be used, examples of which include VERO and HeLa cells, CHO cell lines, and W138, BHK, COS-7, MOP and MDCK cell lines. Promoters suitably selected from known promoters can be used depending on the host. Examples of said promoters that can be used include β-lactamase and lactose promoter systems, trp, tac and T₇ promoter systems, as well as promoters PGK, PHO5, SV-40, CMV, AML and DHFR.

Culturing can be performed under conventional conditions using routinely used media depending on the type of host. Human caldecrin can be recovered from the culture by isolation and purification in accordance with conventional methods such as high-performance liquid chromatography or affinity chromatography.

Thus, rat caldecrin comprising the amino acid sequence from the amino acid Val at position 1 to amino acid Leu at position 239 in SEQ ID NO: 1, or its precursor of the present invention, and human caldecrin comprising the amino acid sequence from the amino acid Val at position 1 to the amino acid Leu at position 239 of SEQ ID NO: 2, or its precursor of the present invention can be obtained.

Since the recombinant human caldecrin of the present invention is a novel substance exhibiting the effect of lowering serum calcium concentration that has increased as a result of accelerated bone resorption in vivo, it is expected to be useful as an active ingredient in drugs for the treatment and prevention of various bone diseases; for example, osteoporosis, primary or secondary hyperparathyroidism, and hypercalcemia due to malignant tumors. The serum calcium-lowering factor of the present invention can be administered parenterally by, for example, intravenous, subcutaneous or intramuscular administration, or parenterally or orally by preparing a formulation in suitable combination with pharmaceutically acceptable carriers typically used in protein formulations; for example, physiological saline, polyethylene glycol, glycerol, gelatin, starch, dextrin and carrier proteins (such as serum albumin).

Use of the DNA of the present invention enables caldecrin to be produced in large amount at high efficiency. The caldecrin thus obtained is useful for treating and preventing various diseases requiring lowering of serum calcium as well as various bone diseases caused by excessively accelerated bone resorption.

### EXAMPLES

Next, the following provides a more detailed explanation of the present invention through Examples.

### Example 1. Cloning of Rat Caldecrin cDNA

In order to clone cDNA coding for rat caldecrin, rabbits were immunized using pig pancreatic caldecrin as an antigen to obtain an antiserum which was then used to perform an immunoscreening of a rat pancreatic λgt11 cDNA library (Clontech) comprising 80,000 λ phages, by which procedure three positive clones were obtained. As a result of preparing lysogens of these positive clones, synthesizing fusion proteins and investigating their reactivity with the antibody, a single positive clone RPC18 was obtained. Moreover, a full length cDNA clone was obtained by again performing screening on the library using a 5' fragment from the PstI site of RPC18 as a probe.

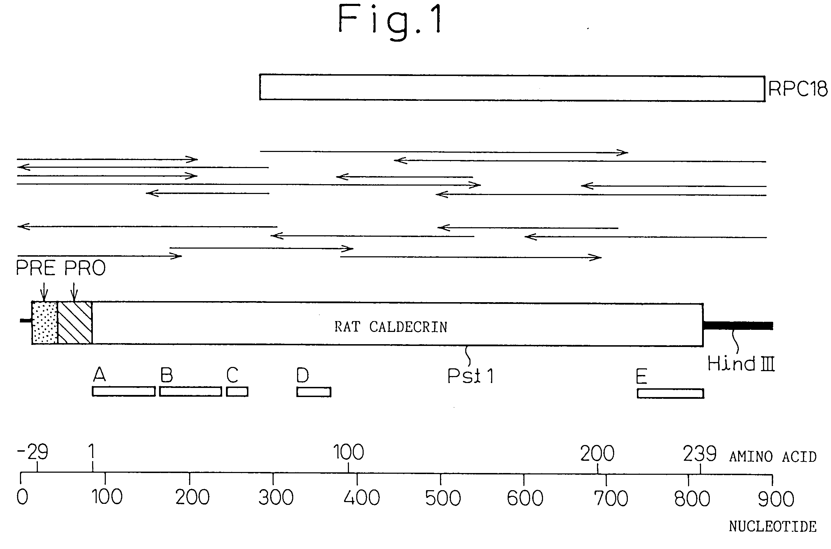

DNA was prepared from the resulting λ phage clone and digested with EcoRI, and the resulting fragment was inserted into the EcoRI site of pUC19. After transfecting E. coli JM109 with this plasmid to amplify the plasmid, the entire DNA sequence was determined as shown in Fig. 1 by cycle sequencing. The total length of this clone is 899 bp, and the amino acid sequence translated from this cDNA encodes 268 amino acids, which have a high degree of homology with known serine proteases including elastases, and contain histidine, aspartic acid and serine residues characteristic to the active site of the serine proteases, and includes a pre-sequence and pro-sequence, in 29 residues of the amino terminus. In addition, as shown in Example 2, all of the partial amino acid sequences determined by analysis of the fragments produced by enzyme digestion of rat pancreatic caldecrin were included, strongly suggesting that the isolated cDNA corresponds to the cDNA of rat caldecrin.

Escherichia coli transformed with the vector pCAGGS-Rat Caldecrin into which the above-mentioned cDNA had been inserted was deposited as JM-109 (pCAGGS-Rat Caldecrin) at the National Institute of Bioscience and Human Technology Agency of Industrial Science and Technology, as FERM BP-4717 in accordance with the Budapest Treaty on June 22, 1994.

### Example 2. Comparison of Rat Pancreatic Caldecrin and cDNA

In order to confirm that the cDNA obtained as described in Example 1 is a cDNA for rat caldecrin, the amino acid sequence predicted from the cDNA and the amino acid sequence of caldecrin isolated and purified from a rat pancreas extract were compared as described below. The rat pancreatic caldecrin was digested with metalloendopeptidase of Grifola frondosa origin in the presence of PMSF, and the resulting peptide fragments were subjected to C18 reverse phase column chromatography.

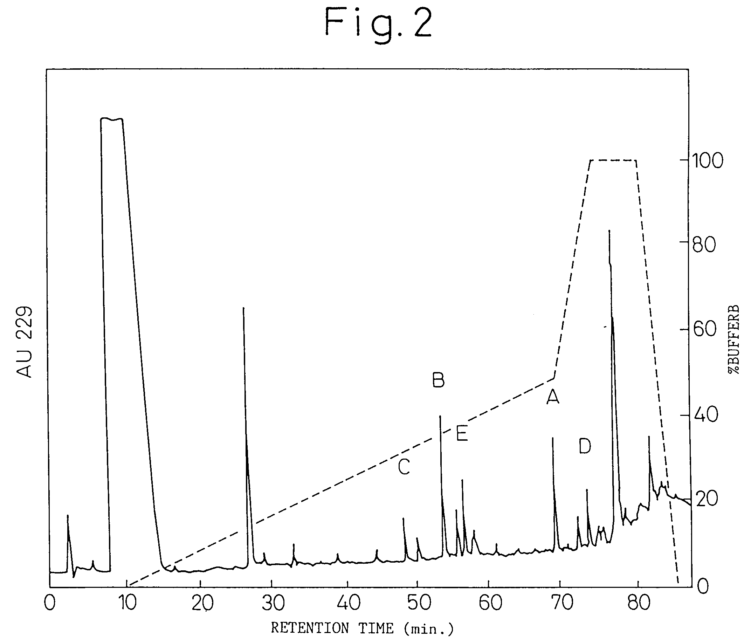

When eluted using a concentration gradient of 80% acetonitrile containing 0.1% trifluoroacetic acid (buffer B), the elution profile shown in Fig. 2 was obtained. Peaks A, B, C, D and E in the drawing were recovered, and amino acid sequences were determined by Edman degradation in accordance with routine methods. The amino acid sequences of all of these fragments were present in the amino acid sequence predicted from the above-mentioned cDNA.

### Example 3. Insertion of Rat Caldecrin cDNA into Mammalian Cell Expression Vector

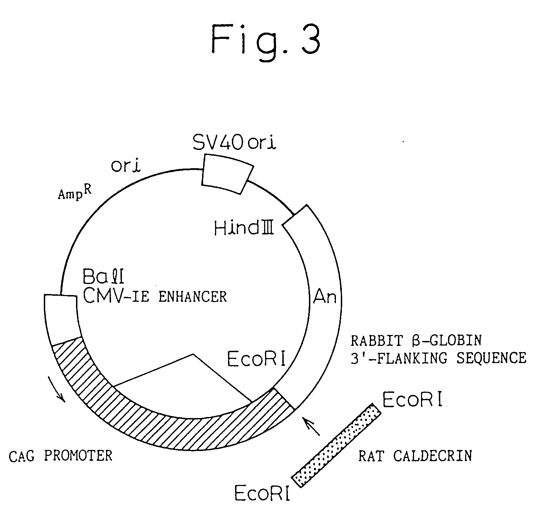

Expression vector pCAGGS developed by Miyazaki et al. was used as a mammalian cell expression vector. This expression vector contains an SV40 replication origin (SV40 Ori), a cytomegalovirus initial enhancer (CMV-IE enhancer), a chicken β-actin promoter (CAG promoter), and a rabbit β-globin polyadenylation signal (An) and a 3'-flanking sequence, and is based on pUC13. As shown in Fig. 3, the rat caldecrin cDNA inserted into pUC19 and excised with EcoRI from the plasmid, was inserted into the EcoRI site of pCAGGS.

### Example 4. Expression of Rat Caldecrin in Mammalian Cells

BMT-10 cells developed by Gerard, R.D. were used as mammalian cells. These cells are immortalized cells prepared by transfecting African green monkey kidney cell strain BSC-1 with plasmid pK1d126 from which the SV40 replication origin had been removed and the SV40 promoter had been replaced with a metallothionein promoter, and induce T antigens in the presence of heavy metals. Introduction of pCAGGS-Rat Caldecrin into BMT-10 cells was performed using the DEAE-dextran method, and transient expression was observed.

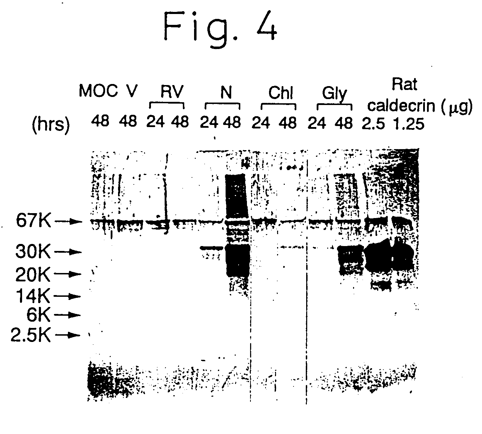

Following transfection, the cells and culture supernatant (1 ml) were applied to SDS-PAGE. When Western blotting was performed, rat caldecrin (N) was demonstrated to be expressed in the cells over time and secreted into the medium, as shown in Fig. 4. In the case of not using a vector (MOC), using a vector only (V), or of inserting caldecrin cDNA in the expression vector in the reverse direction (RV), caldecrin was not observed in the cells or culture supernatant. In addition, although glycerol treatment (Gly) following transfection had no effect on the expression, chlorokine treatment (Chl) was required for the expression.

Note that animal cell BMT-10 used in the present invention was internationally deposited in accordance with the Budapest treaty on June 22, 1994 as FERM BP-4716 at the National Institute of Bioengineering and Human Technology Agency of Industrial Science and Technology.

### Example 5. Anti-PTH Activity in Culture Supernatant

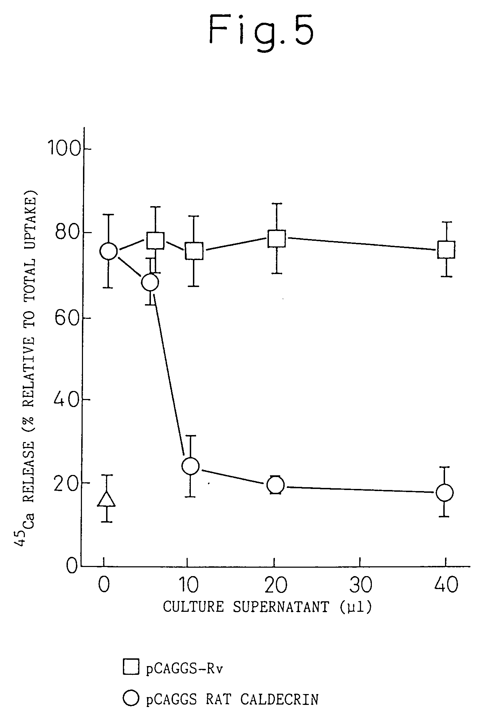

Using the culture supernatant 3 days after transfection of BMT-10 cells with rat caldecrin expression vector (pCAGGS-Rat Caldecrin) or the vector in which cDNA had been inserted in the reverse direction (pCAGGS-RV), the anti-PTH activity in the culture was studied using a fetal mouse bone culture system. As shown in Fig. 5, the culture supernatant from transfection with rat caldecrin expression vector volume-dependently inhibited the liberation of ⁴⁵Ca release by PTH.

On the other hand, inhibitory activity was not observed in the culture supernatant following transfection with pCAGGS-RV. On the basis of the above, recombinant rat caldecrin was confirmed to have activity similar to rat pancreatic caldecrin, thus indicating that the cDNA of the present invention is rat caldecrin cDNA.

### Example 6. Analysis of the Partial Sequence of Pig Pancreatic Caldecrin

In order to determine the caldecrin-specific amino acid sequence, pig pancreatic caldecrin was enzymatically digested and its partial sequences were analyzed. Pig pancreatic caldecrin was treated with metalloendopeptidase of Grifola frondosa origin in the presence of PMSF, and the resulting peptide fragments were subjected to C18 reverse phase column chromatography.

When eluted using a concentration gradient of 80% acetonitrile containing 0.1% trifluoroacetic acid (buffer B), the elution profile shown in Fig. 6 was obtained. Fractions of peaks 1 to 9 in the drawing were selected, and their amino acid sequences were determined as shown in Fig. 7 by Edman degradation in accordance with routine methods. Peak 8 comprised a mixture of two components, and the amino acid sequence of each was determined by continuous comparison of the ratios of phenylthiohydantoinated amino acids present at each stage of the Edman degradation.

### Example 7. Cloning of Human Caldecrin cDNA Fragment by PCR

As shown in Fig. 7, a comparison of the amino acid sequences of rat and pig caldecrin and human elastase revealed a caldecrin-specific sequence. Mixed oligonucleotides comprising 2⁷ oligonucleotides corresponding to this amino acid sequence, to which an EcoRI cleavage sequence was added at the 5' terminal end, and mixed oligonucleotides comprising 2² complementary DNA sequences common to elastase and rat caldecrin cDNA, to which an EcoRI cleavage sequence was added at the 5' terminal end, were synthesized. Using these oligonucleotides as primers, PCR was performed with Taq polymerase in accordance with routine methods using human pancreatic cDNA (Clontech, QUICK-Clone™ cDNA) as a template. The reaction mixture was applied to agarose gel electrophoresis, and the resulting approximately 200 bp band was extracted from the gel. The resulting DNA was digested with EcoRI and cloned in pUC19 plasmid vector.

### Example 8. Cloning of Human Caldecrin cDNA

The above-mentioned human caldecrin cDNA fragment was cleaved from pUC19 containing said human caldecrin cDNA fragment by digestion with EcoRI, and the cDNA fragment was labeled by the random primer method using [α-³²P]dCTP to prepare a probe. Hybridization screening was then performed with a human pancreatic λgt11cDNA library (Clontech) using this probe. λgt11 was infected into host E. coli Y1090r⁻ and cultured by inoculating onto a plate at 20,000 plaques/plate (20 cm x 10 cm). A replica was prepared by transferring the plaques to a Nylon membrane filter (Amersham, Hybond™-N⁺). After alkaline denaturation and neutralization, the DNA was fixed by UV irradiation.

This membrane filter was incubated for 16 hours at 42°C in a hybridization solution containing roughly 10,000 cpm of ³²P-labeled probe (containing 50 ml of formamide, 25 ml of 20x SSC, 10 ml of 50x Denhardt's solution, 1 ml of 10% SDS and 1 ml of 10 mg/ml denatured salmon sperm DNA in 100 ml). The filter was then washed with 2x SSC containing 0.1% SDS at room temperature for 2 hours and then at 65°C for 30 minutes.

A plurality of clones that hybridized to the DNA probe used were selected by autoradiography of this filter. Similar hybridization was again repeated on each clone, and λgt11 clones were isolated into single clones. Each λgt11 clone was digested with EcoRI, and the resulting fragments were cloned into pUC19, and their sequences were subsequently analysed. Analysis of DNA sequences was performed by applying the cycle sequence reaction with Tag polymerase using a DNA sequencer (Perkin-Elmer, ABI373).

### Example 9. Production of Recombinant Human Caldecrin Using a Baculovirus Expression System

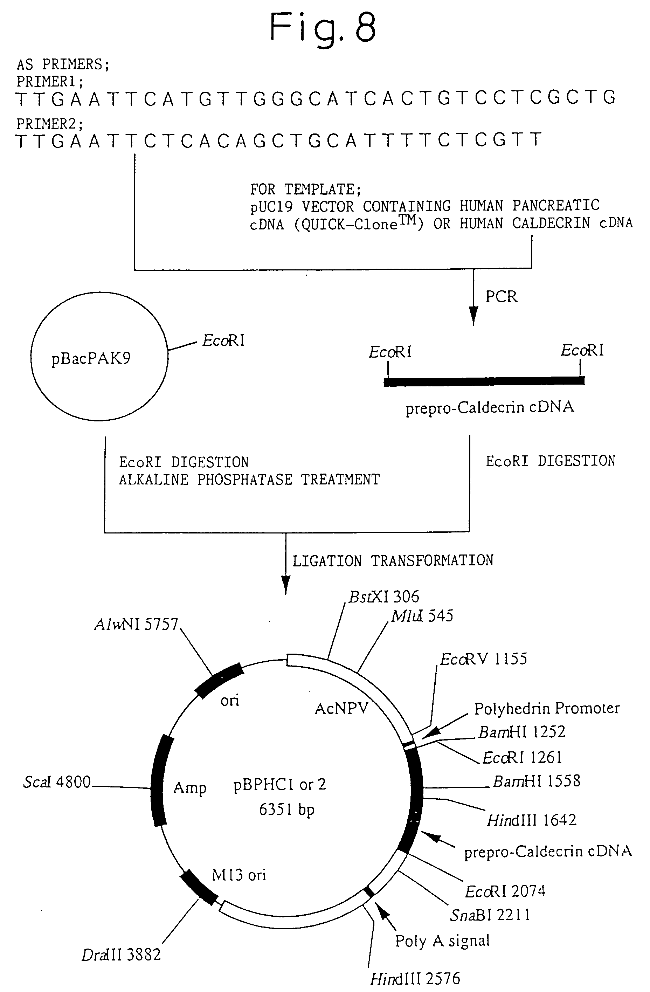

Production of recombinant human caldecrin was performed using the Autographa californica (AcMNPV) Baculovirus expression system (Clontech, BacPCK™). As shown in Fig. 8, in order to remove the non-coding regions at the 3' and 5' terminal ends, a pair of oligonucleotides containing an EcoRI recognition sequence were used for the primer for the 5' terminal end. The DNA from the start codon to the stop codon of human caldecrin cDNA was amplified by PCR using pUC19 containing a human pancreatic cDNA library (QUICK-Clone™) or the cloned human caldecrin cDNA as template, and inserted into the EcoRI site of the transfer vector pBacPAK9.

The resulting transfer vectors pBPHC1 (N = C in SEQ ID NO: 2) and pBPHC2 (N = T in SEQ ID NO: 2) were each replicated by introduction into E. coli DH5α to obtain a large amount of plasmid DNA, which was then purified by cesium chloride density gradient centrifugation. After introducing pBPHC1 or pBPHC2 into Sf9 cells with the viral DNA BacPAK6 (digested with Bsu36I) using lipofectin™ (Gibco-BRL), the cells were cultured for 3 days at 27°C in TNM-FH insect cell medium containing 10% fetal bovine serum. The supernatant of this culture was used as a virus stock.

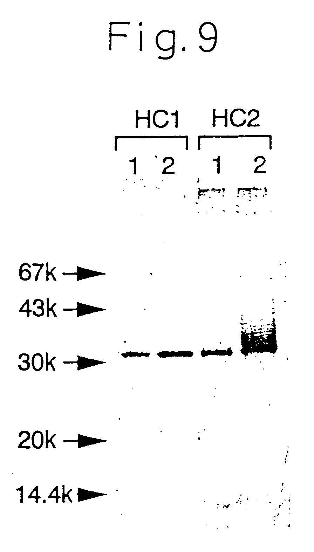

For the expression of recombinant human caldecrin, the stock liquid was added to Sf9 cells and cultured for 3 days at 27°C in TNM-FH medium not containing serum, and after that the supernatant was recovered. The culture supernatant was applied to SDS-PAGE, and Western blotting was performed using an antiserum prepared by immunizing a rabbit with pig pancreatic caldecrin so as to confirm the expression of roughly 30 kD human procaldecrin. The result is shown in Fig. 9.

### Example 10. Purification of Recombinant Human Caldecrin and its Serum Calcium-Lowering Effect

The purification and activity study described below was performed using the expression culture supernatant of the recombinant human caldecrin HC2 obtained in Example 9.

The culture supernatant was concentrated 10 times with an ultrafiltration membrane having a fractionated molecular weight cut-off of 10,000 (Amicon), and the solvent was replaced with 10 mM sodium phosphate buffer (pH 6.8) by Sephadex G-25 (Pharmacia, PD-10) or by dialysis. This solution was adsorbed onto an hydroxyapatite column (BIO-RAD, Bio-Scale CHT-1). After thoroughly washing with 10% and 20% solutions of 500 mM sodium phosphate buffer (pH 6.8), the column was eluted with a 30% solution of the same to obtain procaldecrin as a symmetrical single peak. Subsequently, protease activity was measured in the following manner for each of the trypsin-treated and trypsin-untreated fractions. Specially, after adding trypsin (20 µg/ml) to 15 µl of each fraction and incubating it for 15 minutes at 25°C, 200 µl of 0.1 mM succinyl-Ala-Ala-Pro-Phe-p-nitroanilide dissolved in 0.1 M Tris-HCl buffer (pH 7.8) and 10 mM CaCl₂ were added and allowed to react for 5 minutes. The reaction was stopped by adding 30 µl of 36% acetic acid, following with absorbance was measured at 415 nm. Protease activity was only detected in the fraction eluted with the 30% solution of 500 mM sodium phosphate. Protease activity was not detected in any of the fractions not treated with trypsin. Based on these findings, it was clear that the resulting caldecrin fraction was the pro form. In addition, the HC1 expression culture supernatant obtained in Example 9 was also similarly purified using the same method as for HC2. Although a single peak was not obtained, a partially purified procaldecrin fraction was obtained using protease activity as an indicator.

Western blotting following SDS-BOGE was performed in the same manner as described in Example 9 on the above-mentioned HC1 and HC2 procaldecrin fractions. Approximately 30 kD bands were confirmed to react with anti-pig caldecrin antiserum.

Procaldecrin was converted to its mature form by treating it for 30 minutes at 25°C following the addition of 1 µl of 2 mg/ml trypsin solution to 1 ml of purified procaldecrin solution obtained as described above. A mixture to which this solution, protease inhibitor and 20 µl of 50 mM phenylmethanesulfonylfluoride (PMSF) were added was substituted with phosphate-buffered saline. This solution was administered into the caudal vein of 4 to 5 week old Balb/c male mice, starved from the previous day, at a ratio of 125 µg of total protein per kg body weight. Blood samples were drawn from the heart 2 hours later to obtain serum. Serum calcium concentration was measured using the Merck Auto Ca Kit. The results were as shown below. Furthermore, the control group was administered phosphate-buffered saline alone.

| | Serum calcium concentration (mg/dl) | Level of significance relative to control group (t test) |
|---|---|---|
| Control | 10.15±0.186 | |
| HC1 (non-PMSF treated) | 9.52±0.196 | <0.05 |
| HC1 (PMSF treated) | 9.26±0.205 | <0.01 |
| HC2 (non-PMSF treated) | 9.22±0.260 | <0.01 |
| HC2 (PMSF treated) | 9.08±0.171 | <0.01 |

Note that E. coli DH5α, into which the above-mentioned vector pBPHC1 had been introduced, was named Escherichia coli DH5α (pBPHC1), and was internationally deposited in accordance with the Budapest treaty as FERM BP-4978 on January 25, 1995 at the National Institute of Bioscience and Human Technology Agency of Industrial Science and Technology. In addition, E. coli DH5α, into which the above-mentioned vector pBHC2 had been introduced, was named Escherichia coli DH5α (pBPHC2), and was also internationally deposited in accordance with the Budapest treaty as FERM BP-4979 on January 25, 1995 at the National Institute of Bioscience and Human Technology Agency of Industrial Science and Technology.

### Reference to Microorganisms Deposited Under Regulation 13 bis of the Regulations

Depositing Agency: National Institute of Bioscience and Human Technology Agency of Industrial Science and Technology, Ministry of International Trade and Industry

Address: 1-1-3 Higashi, Tsukuba, Ibaraki, Japan

Deposition Numbers and Deposition Dates:
1. BMT-10
   Deposition No.: FERM BP-4716
   Deposition Date: June 22, 1994
2. Escherichia coli DH5α (pBPHC1)
   Deposition No.: FERM BP-4978
   Deposition Date: January 25, 1995
3. Escherichia coli DH5α (pBPHC2)
   Deposition No.: FERM BP-4979
   Deposition Date: January 25, 1995

SEQUENCE LISTING

SEQ ID NO: 1
Sequence Length: 807
Sequence Type: Nucleic acid
Strandedness: Double strand
Topology: Linear
Molecular Type: cDNA to mRNA
Source:
Organism: rat
Strain:
Characteristic of sequence:
Amino acid number -29 to -14 Pre-sequence
Amino acid number -13 to -1 Pro-sequence
Amino acid number 1 to 239 Mature sequence
Sequence:
ATG TTG GGA ATT ACG GTC CTC GCT GCC ATC CTG GCC TGC GCC TCT TGC 48
Met Leu Gly Ile Thr Val Leu Ala Ala Ile Leu Ala Cys Ala Ser Cys
-29 -25 -20 -15
TGC GGG AAC CCC GCC TTC CCA CCT AAC CTG TCA ACC AGA GTG GTA GGA 96
Cys Gly Asn Pro Ala Phe Pro Pro Asn Leu Ser Thr Arg Val Val Gly
-10 -5 -1 1
GGA GAG GAT GCT GTC CCC AAC AGC TGG CCT TGG CAG GTC TCT CTC CAG 144
Gly Glu Asp Ala Val Pro Asn Ser Trp Pro Trp Gln Val Ser Leu Gln
                5                   10                  15
TAC CTC AAG GAC GAC ACA TGG AGG CAC ACC TGT GGG GGA AGT CTC ATC 192
Tyr Leu Lys Asp Asp Thr Trp Arg His Thr Cys Gly Gly Ser Leu Ile
20 25 30 35
ACC ACC AGC CAC GTC CTC ACT GCC GCC CAC TGC ATC AAC AAA GAC TTC 240
Thr Thr Ser His Val Leu Thr Ala Ala His Cys Ile Asn Lys Asp Phe
40 45 50
ACT TAC CGT GTG GGC CTG GGG AAG TAT AAT CTG ACA GTG GAG GAT GAG 288
Thr Tyr Arg Val Gly Leu Gly Lys Tyr Asn Leu Thr Val Glu Asp Glu
55 60 65

GAA GGC TCC GTG TAC GCT GAG GTG GAC ACC ATC TAC GTC CAT GAG AAG 336
Glu Gly Ser Val Tyr Ala Glu Val Asp Thr Ile Tyr Val His Glu Lys
70 75 80
TGG AAC CGA CTC TTC CTG TGG AAC GAC ATC GCT ATC ATT AAG TTG GCT 384
Trp Asn Arg Leu Phe Leu Trp Asn Asp Ile Ala Ile Ile Lys Leu Ala
                85                  90                  95
GAG CCT GTG GAA CTG AGC AAC ACC ATC CAG GTG GCC TGC ATC CCA GAG 432
Glu Pro Val Glu Leu Ser Asn Thr Ile Gln Val Ala Cys Ile Pro Glu
100 105 110 115
GAA GGT TCC CTG CTG CCT CAG GAC TAT CCC TGC TAT GTC ACG GGC TGG 480
Glu Gly Ser Leu Leu Pro Gln Asp Tyr Pro Cys Tyr Val Thr Gly Trp
120 125 130
GGT CGC CTC TGG ACC AAT GGT CCC ATC GCT GAA GTG CTC CAG CAG GGC 528
Gly Arg Leu Trp Thr Asn Gly Pro Ile Ala Glu Val Leu Gln Gln Gly
135 140 145
CTG CAG CCC ATC GTG AGC CAT GCC ACG TGC TCC AGG TTG GAC TGG TGG 576
Leu Gln Pro Ile Val Ser His Ala Thr Cys Ser Arg Leu Asp Trp Trp
150 155 160
TTC ATC AAG GTC CGG AAG ACG ATG GTG TGC GCT GGG GGT GAT GGC GTC 624
Phe Ile Lys Val Arg Lys Thr Met Val Cys Ala Gly Gly Asp Gly Val
                165                 170                 175
ATC TCT GCC TGT AAC GGA GAT TCT GGC GGC CCA CTG AAC TGC CAA GCA 672
Ile Ser Ala Cys Asn Gly Asp Ser Gly Gly Pro Leu Asn Cys Gln Ala
180 185 190 195
GAA GAC GGC TCA TGG CAG GTG CAC GGC ATC GTG AGC TTC GGT TCC AGT 720
Glu Asp Gly Ser Trp Gln Val His Gly Ile Val Ser Phe Gly Ser Ser
200 205 210
AGC GGC TGC AAC GTA CAC AAG AAA CCG GTA GTC TTC ACC CGA GTG TCT 768
Ser Gly Cys Asn Val His Lys Lys Pro Val Val Phe Thr Arg Val Ser
215 220 225
GCC TAC AAT GAC TGG ATC AAC GAG AAA ATA CAA CTG TGA 807
Ala Tyr Asn Asp Trp Ile Asn Glu Lys Ile Gln Leu ***
230 235
SEQ ID NO: 2
Sequence Length: 807

Sequence Type: Nucleic acid
Strandedness: Double strand
Topology: Linear
Molecular Type: cDNA to mRNA
Source:
Organism: Human
Strain:
Characteristic of sequence:
Amino acid number -29 to -14 Pre-sequence
Amino acid number -13 to -1 Pro-sequence
Amino acid number 1 to 239 Mature sequence
N represents T or C; and Xaa represents Trp or Arg.
Sequence:
ATG TTG GGC ATC ACT GTC CTC GCT GCG CTC TTG GCC TGT GCC TCC AGC 48
Met Leu Gly Ile Thr Val Leu Ala Ala Leu Leu Ala Cys Ala Ser Ser
-29 -25 -20 -15
TGT GGG GTG CCC AGC TTC CCG CCC AAC CTA TCC GCC CGA GTG GTG GGA 96
Cys Gly Val Pro Ser Phe Pro Pro Asn Leu Ser Ala Arg Val Val Gly
-10 -5 -1 1
GGA GAG GAT GCC CGG CCC CAC AGC TGG CCC TGG CAG ATC TCC CTC CAG 144
Gly Glu Asp Ala Arg Pro His Ser Trp Pro Trp Gln Ile Ser Leu Gln
                5                   10                  15
TAC CTC AAG AAC GAC ACG TGG AGG CAT ACG TGT GGC GGG ACT TTG ATT 192
Tyr Leu Lys Asn Asp Thr Trp Arg His Thr Cys Gly Gly Thr Leu Ile
20 25 30 35
GCT AGC AAC TTC GTC CTC ACT GCC GCC CAC TGC ATC AGC AAC ACC NGG 240
Ala Ser Asn Phe Val Leu Thr Ala Ala His Cys Ile Ser Asn Thr Xaa
40 45 50
ACC TAC CGT GTG GCC GTG GGA AAG AAC AAC CTG GAG GTG GAA GAC GAA 288
Thr Tyr Arg Val Ala Val Gly Lys Asn Asn Leu Glu Val Glu Asp Glu
55 60 65
GAA GGA TCC CTG TTT GTG GGT GTG GAC ACC ATC CAC GTC CAC AAG AGA 336
Glu Gly Ser Leu Phe Val Gly Val Asp Thr Ile His Val His Lys Arg
70 75 80

TGG AAT GCC CTC CTG TTG CGC AAT GAT ATT GCC CTC ATC AAG CTT GCA 384
Trp Asn Ala Leu Leu Leu Arg Asn Asp Ile Ala Leu Ile Lys Leu Ala
                85                  90                  95
GAG CAT GTG GAG CTG AGT GAC ACC ATC CAG GTG GCC TGC CTG CCA GAG 432
Glu His Val Glu Leu Ser Asp Thr Ile Gln Val Ala Cys Leu Pro Glu
100 105 110 115
AAG GAC TCC CTG CTC CCC AAG GAC TAC CCC TGC TAT GTC ACC GGC TGG 480
Lys Asp Ser Leu Leu Pro Lys Asp Tyr Pro Cys Tyr Val Thr Gly Trp
120 125 130
GGC CGC CTC TGG ACC AAC GGC CCC ATT GCT GAT AAG CTG CAG CAG GGC 528
Gly Arg Leu Trp Thr Asn Gly Pro Ile Ala Asp Lys Leu Gln Gln Gly
135 140 145
CTG CAG CCC GTG GTG GAT CAC GCC ACG TGC TCC AGG ATT GAC TGG TGG 576
Leu Gln Pro Val Val Asp His Ala Thr Cys Ser Arg Ile Asp Trp Trp
150 155 160
GGC TTC AGG GTG AAG AAA ACC ATG GTG TGC GCT GGG GGC GAT GGC GTC 624
Gly Phe Arg Val Lys Lys Thr Met Val Cys Ala Gly Gly Asp Gly Val
                165                 170                 175
ATC TCA GCC TGC AAT GGG GAC TCC GGT GGC CCA CTG AAC TGC CAG TTG 672
Ile Ser Ala Cys Asn Gly Asp Ser Gly Gly Pro Leu Asn Cys Gln Leu
180 185 190 195
GAG AAC GGT TCC TGG GAG GTG TTT GGC ATC GTC AGC TTT GGC TCC CGG 720
Glu Asn Gly Ser Trp Glu Val Phe Gly Ile Val Ser Phe Gly Ser Arg
200 205 210
CGG GGC TGC AAC ACC CGC AAG AAG CCG GTA GTC TAC ACC CGG GTG TCC 768
Arg Gly Cys Asn Thr Arg Lys Lys Pro Val Val Tyr Thr Arg Val Ser
215 220 225
GCC TAC ATC GAC TGG ATC AAC GAG AAA ATG CAG CTG TGA 807
Ala Tyr Ile Asp Trp Ile Asn Glu Lys Met Gln Leu ***
230 235

## Claims

1. A DNA coding for rat caldecrin comprising an amino acid sequence from the amino acid Val at position 1 to the amino acid Leu at position 239 in SEQ ID NO: 1, or its precursor.

2. A DNA coding for rat caldecrin having the amino acid sequence from the amino acid Val at position 1 to the amino acid Leu at position 239 in SEQ ID NO: 1.

3. A DNA coding for a rat caldecrin precursor having the amino acid sequence from the amino acid Met at position -29 to the amino acid Leu at position 239 in SEQ ID NO: 1.

4. A DNA according to claim 1, comprising the nucleotide sequence from the nucleotide G at position 88 to the nucleotide G at position 804 in SEQ ID NO: 1.

5. A DNA according to claim 2, consisting of the nucleotide sequence from the nucleotide G at position 88 to the nucleotide G at position 804 in SEQ ID NO: 1.

6. A DNA according to claim 3, consisting of the nucleotide sequence from the nucleotide A at position 1 to the nucleotide G at position 804 in SEQ ID NO: 1.

7. An expression vector containing a DNA according to any one of claims 1 to 6.

8. A host cell transformed with an expression vector according to claim 7.

9. A process for producing rat caldecrin or its precursor characterized in that host cells according to claim 8 are cultured and rat caldecrin or its precursor is removed from said culture.

10. A rat caldecrin precursor containing the amino acid sequence from the amino acid Val at position 1 to the amino acid Leu at position 239 in SEQ ID NO: 1.

11. A DNA coding for human caldecrin comprising the amino acid sequence from the amino acid Val at position 1 to the amino acid Leu at position 239 in SEQ ID NO: 2, or its precursor.

12. A DNA coding for human caldecrin having the amino acid sequence from the amino acid Val at position 1 to the amino acid Leu at position 239 in SEQ ID NO: 2.

13. A DNA coding for a human caldecrin precursor having the amino acid sequence from the amino acid Met at position -29 to the amino acid Leu at position 239 in SEQ ID NO: 2.

14. A DNA according to claim 11, comprising the nucleotide sequence from the nucleotide G at position 88 to the nucleotide G at position 804 in SEQ ID NO: 2.

15. A DNA according to claim 12, consisting of the nucleotide sequence from the nucleotide G at position 88 to the nucleotide G at position 804 in SEQ ID NO: 2.

16. A DNA according to claim 13, consisting of the nucleotide sequence from the nucleotide A at position 1 to the nucleotide G at position 804 in SEQ ID NO: 2.

17. A DNA within a human cDNA library or genome library, that hybridizes to DNA comprising a portion or the entirety of a nucleotide sequence corresponding to the amino acid sequence from the amino acid Glu at position 63 to the amino acid Trp at position 131 in SEQ ID NO: 2.

18. An expression vector containing DNA according to any one of claims 11 to 17.

19. A host cell transformed with an expression vector according to claim 18.

20. A process for producing human caldecrin or its precursor characterized in that host cells according to claim 19 are cultured and human caldecrin or its precursor is obtained from said culture.

21. A human caldecrin precursor containing the amino acid sequence from the amino acid Val at position 1 to the amino acid Leu at position 239 in SEQ ID NO: 2.

22. A serum calcium-lowering agent comprising rat caldecrin or human caldecrin and a pharmacologically acceptable carrier.

23. A serum calcium-lowering agent according to claim 22 wherein said rat caldecrin has the amino acid sequence from Val of amino acid no. 1 to Leu of amino acid no. 239 shown in SEQ ID NO: 1, and said human caldecrin has the amino acid sequence from Val of amino acid no. 1 to Leu of amino acid 239 shown in SEQ ID NO: 2.

24. A DNA that hybridizes to a DNA according to any one of claims 1 to 6 and codes for a protein that has a serum calcium-lowering action.

25. A DNA that hybridizes to a DNA according to any one of claims 11 to 16 and codes for a protein that has a serum calcium-lowering action.

26. A human caldecrin consisting of at least a part of the amino acid sequence shown in SEQ ID NO: 2.
